# EUROPEAN PATENT SPECIFICATION

(11) **EP 4 274 550 B1**
(45) Date of publication and mention of the grant of the patent: **20.08.2025**
(21) Application number: 22701700.1
(22) Date of filing: 04.01.2022
(51) Int. Cl.: A61K 9/00, A61P 25/00, A61P 35/00, A61N 1/32, A61N 1/40, A61N 1/36

(54) **DEVICE AND ANTITUMOR DRUG FOR THE TREATMENT OF NEOPLASTIC CELLS**
VORRICHTUNG UND ANTITUMORMITTEL ZUR BEHANDLUNG VON NEOPLASTISCHEN ZELLEN
DISPOSITIF ET MÉDICAMENT ANTITUMORAL POUR LE TRAITEMENT DE CELLULES NÉOPLASIQUES

(30) Priority: 05.01.2021 IT 202100000119
(43) Date of publication of application: 15.11.2023
(73) Proprietor: TELEA ONCOLOGY RESEARCH S.R.L., 36066 Sandrigo (VI) (IT); Azienda Ulss 8 Berica, 36100 Vicenza (IT)
(72) Inventor: POZZATO, Gianantonio, 36100 Vicenza (IT); POZZATO, Alessandro, 36100 Vicenza (IT); ASTORI, Giuseppe, 36100 Vicenza (IT); CATANZARO, Daniela, 36040 Torri Di Quartesolo (VI) (IT); VOLPIN, Lorenzo, 35123 Padova (IT); RANERI, Fabio, 36100 Vicenza (IT)
(74) Representative: Marchioro, Paolo
(86) International application number: PCT/IB2022/050042
(87) International publication number: WO 2022/149061

(56) References cited:
- EP-A1- 3 439 733
- EP-B1- 2 092 956
- EP-B1- 3 439 733
- WO-A1-2019/100016
- US-A1- 2007 239 213
- US-A1- 2017 209 579
- US-A1- 2020 009 377
- US-A1- 2020 269 041

## Description

The present invention is set out in the appended claims. The present disclosure generally relates to a device configured to block or inhibit the growth of neoplastic cells in a target region of a living organism, particularly the use of a device for blocking or inhibiting the growth of neoplastic cells in combination with an antitumor drug.

Neoplasms or tumors originate from alteration of cells that multiply and spread throughout the body, escaping regulatory mechanisms. There are numerous types of neoplasms that can affect substantially any organ. They are usually classified into benign or malignant according to the ability of the neoplastic cells to invade surrounding structures and more distant organs. In addition to tumors of solid organs, the term neoplasm includes also tumors of blood cells, such as lymphomas and leukaemias.

Neoplasms continue to be among the leading causes of death and deterioration in quality of life despite the major successes in the clinical field achieved in recent decades thanks to targeted prevention measures, early diagnosis and therapy.

Tumor therapy is essentially based on surgery, especially in localised forms, radiotherapy and drugs.

Glioblastoma multiforme (GBM) represents one of the most aggressive tumors for humans due to the ability of GBM neoplastic cells to infiltrate the parenchyma adjacent to the tumor lesion (*DeAngelis et al, 2001*)*.*

GBM patients usually undergo resection, possibly complete, of the lesion by surgery, followed by radiotherapy and chemotherapy with the drug temozolomide, an alkylating antitumor drug (*Alifieris et al, 2015*)*.*

However, despite a multi-pronged therapeutic approach, GBM is characterized by a high recurrence rate, drug resistance and devastating neurological deterioration (*Wilson et al, 2014*)*.*

GBM is a particularly infiltrative tumor, and the performance of a complete surgical resection of the lesion is therefore substantially impossible, so that almost all patients develop a relapse and have a survival time of only 14 months (*Becker et al, 2012*)*.* Additionally, 5 years after surgery, less than 5% of patients are still alive (*Ostrom et al, 2014*)*.*

It is therefore clear how necessary it is to develop new therapeutic strategies to improve the efficacy of the drugs in use and to reduce the corresponding adverse effects, thus improving patients' quality of life.

In this sense, one of the most promising therapies comprises the use of nonionizing electromagnetic fields (EMFs).

This therapy is particularly promising as it appears to have an excellent degree of safety, low toxicity and the possibility of combination with conventional drug therapies (*Mattson et al, 2019*)*.*

In recent years, several EMFs technologies have been tested and have shown to have a good level of efficacy against different types of tumors, both when used alone and in combination with chemotherapy (*Pasi et al, 2016*)*.*

It has been found that different frequency ranges trigger different response mechanisms in the cells that can lead to the reduction of cell proliferation of neoplastic cells by acting on the formation and stability of the mitotic spindle of these cells (*Giladi et al, 2015*)*.*

In 2011, the American Food and Drug Administration approved the use of Tumor Treating Fields (TTFields) for the treatment of patients with recurrent GBM.

Several prior art documents have been published to this effect, such as, by way of example, documents US2020269041 and EP3439733.

### Description

The object of the present description is to realize an alternative device and method to those of a known type, adapted to block or inhibit the growth of neoplastic cells in a target region of a living organism.

In particular, it is an object of the present description that such a device and method may be used in the treatment of neoplasms, even of an aggressive type, such as glioblastoma multiforme.

Still, it is an object of the present description that such method and use of the device in a patient may also be of the non-invasive type.

Furthermore, it is an object of the present description to realize an antitumor drug which has greater therapeutic efficacy and a reduced toxicological profile, improving the patient's quality of life. According to the present invention, and to achieve the objects mentioned above, an antitumor drug as set out in claim 1 is provided.

The present invention also pertains to a device as set forth in claim 6.

Furthermore, the objects are also achieved by a non-claimed method for treating neoplastic cells, according to what will be described below.

In particular, the present description relates to an antitumor drug for use in a method for blocking or inhibiting the growth of neoplastic cells in a target region of a living organism, wherein said method comprises treating said neoplastic cells with the antitumor drug prior to, simultaneously with or subsequent to the application of electric current waves to the target region for a predefined period of time, and wherein the electric current waves have a waveform with a fundamental frequency higher than or equal to 2 MHz.

Preferably, such electric current waves have a waveform with a fundamental frequency ranging between 2 and 64 MHz, more preferably ranging between 2 and 20 MHz, even more preferably ranging between 4 and 20 MHz.

Much more preferably, such electric current waves have a waveform with a fundamental frequency of about 4 MHz.

It is specified that the application of such electric current waves will also be referred to in this document as Quantum Molecular Resonance or QMR.

Furthermore, according to an aspect of the present description, such electric current waves have a sinusoidal waveform.

According to one aspect of the description, this waveform is distorted by the presence of harmonics.

According to another aspect of the present description, the sinusoidal waveform is distorted by the presence of at least second and third order harmonics.

According to one aspect of the description, the treatment of neoplastic cells with the antitumor drug is carried out simultaneously with or subsequent to the application of the aforesaid electric current waves.

Preferably, according to one aspect of the description, the treatment of the neoplastic cells with the antitumor drug is carried out simultaneously with the application of the electric current waves to the target region, while the neoplastic cells are treated with this antitumor drug.

According to one aspect of the description, the aforesaid antitumor drug comprises temozolomide.

According to one aspect of the present description, the neoplastic cells comprise glioblastoma cells.

In particular, the target region belongs to a living organism, preferably a human subject.

The aforesaid target region preferably comprises the central nervous system.

A device for blocking or inhibiting the growth of neoplastic cells in a target region of a living organism is also part of the present description, wherein said device is configured to generate electric current waves having a waveform having a fundamental frequency higher than or equal to 2 MHz, preferably ranging between 2 and 64 MHz, more preferably ranging between 2 and 20 MHz, even more preferably ranging between 4 and 20 MHz, much more preferably about 4 MHz, and wherein said waveform is a sinusoidal waveform distorted by the presence of harmonics.

This device comprises one or more electrodes connected to at least one radiofrequency circuit adapted to supply each of these electrodes with a current wave having a fundamental frequency higher than or equal to 2 MHz. Such one or more electrodes are configured so as to transmit said electric current waves to the aforesaid target region.

According to one aspect of the invention, the electrodes of the device are electrodes of the implantable type, adapted to be implanted at or near the aforesaid target region.

A non-claimed method for blocking or inhibiting the growth of neoplastic cells in a target region is also part of the present description.

This target region preferably belongs to a living organism.

According to one aspect of the method of the present description, it comprises treating the aforesaid neoplastic cells by applying electric current waves to the aforesaid target region for a predefined period of time, wherein said electric current waves have a waveform with a fundamental frequency higher than or equal to 2 MHz, preferably ranging between 2 and 64 MHz, more preferably ranging between 2 and 20 MHz, even more preferably ranging between 4 and 20 MHz, much more preferably about 4 MHz.

Furthermore, according to one aspect of the method of the description, such a waveform is a sinusoidal waveform.

Again, according to one aspect of the description, this sinusoidal waveform is distorted by the presence of harmonics.

Again, according to another aspect of the method of the present description, the sinusoidal waveform is distorted by the presence of at least second and third order harmonics.

Furthermore, according to one aspect of the method of the description, the neoplastic cells comprise glioblastoma cells.

According to one aspect of the description, the target region comprises the central nervous system.

According to a further aspect of the method of the description, this method comprises the following steps:
- connecting a device adapted to generate electric current waves, having a waveform with a fundamental frequency higher than or equal to 2 MHz and preferably having a sinusoidal waveform distorted by the presence of harmonics, to one or more electrodes;
- applying at least one of these electrodes to the surface of the body of the living organism at which the target region comprising the aforesaid neoplastic cells is located;
- activating the device such as to transfer the current waves to said electrode and keeping the device activated for a predefined time.

Alternatively, according to another aspect of the method of the description, this method comprises the following steps:
- connecting a device adapted to generate electric current waves, having a waveform with a fundamental frequency higher than or equal to 2 MHz and preferably having a sinusoidal waveform distorted by the presence of harmonics, to one or more electrodes of the implantable type;
- implanting at least one of these electrodes in the living organism at or near the target region comprising the aforesaid neoplastic cells;
- activating the device such as to transfer the current waves to said electrode and keeping the device activated for a predefined time.

Preferably, the method of the present description, makes use of the device of the present invention.

Further features and advantages of the drug, device and method of the description, will be apparent to one skilled in the art from the following description of preferred embodiments which is given by way of illustration, but not limitation.

### Brief description of the figures

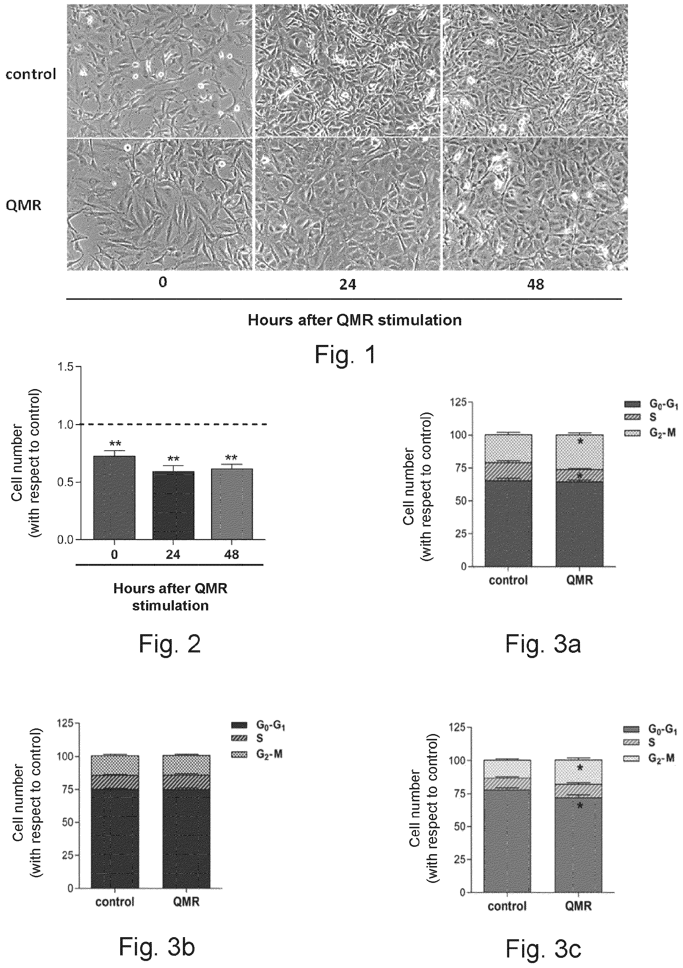

- Figure 1 shows representative images, acquired under an inverted light microscope, of cell cultures of glioblastoma A172 at 0, 24 and 48 hours after the end of the QMR stimulation and untreated control cells (Control); the images were acquired using an Axiovert 40 CFL microscope, 10X magnification;
- Figure 2 shows the cell viability ratio of A172 cells at 0, 24 and 48 hours after the end of the QMR stimulation, compared to control cells (Control) (mean ± SEM of at least 4-5 independent experiments; ** = p <0.01; QMR Vs Control (One-sample t-test));
- Figures 3a, 3b and 3c show the percentages of A172 cells for each phase of the cell cycle (G₀-G₁, S, G₂-M): the progression of the cell cycle was monitored by flow cytometry at 0 (Fig. 3a), 24 (Fig. 3b) and 48 hours (Fig. 3c) after the end of the QMR stimulation (mean ± SEM of at least 4-6 independent experiments; * = p <0.05; QMR Vs Control (Unpaired t-test));

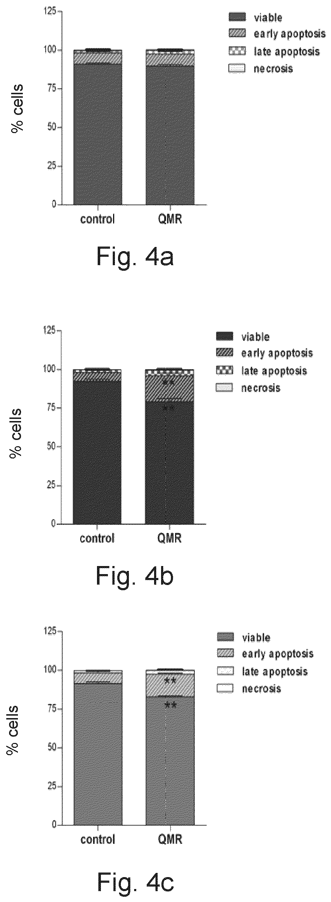

- Figures 4a, 4b and 4c show the induction of apoptosis in A172 cells at 0 (Fig. 4a), 24 (Fig. 4b) and 48 hours (Fig. 4c) after the end of the QMR stimulation; the histograms show the percentage of viable and dead cells, subdividing early apoptosis from late apoptosis and necrosis (mean ± SEM of at least 3-5 independent experiments; ** = p <0.01; QMR Vs Control (Unpaired t-test));

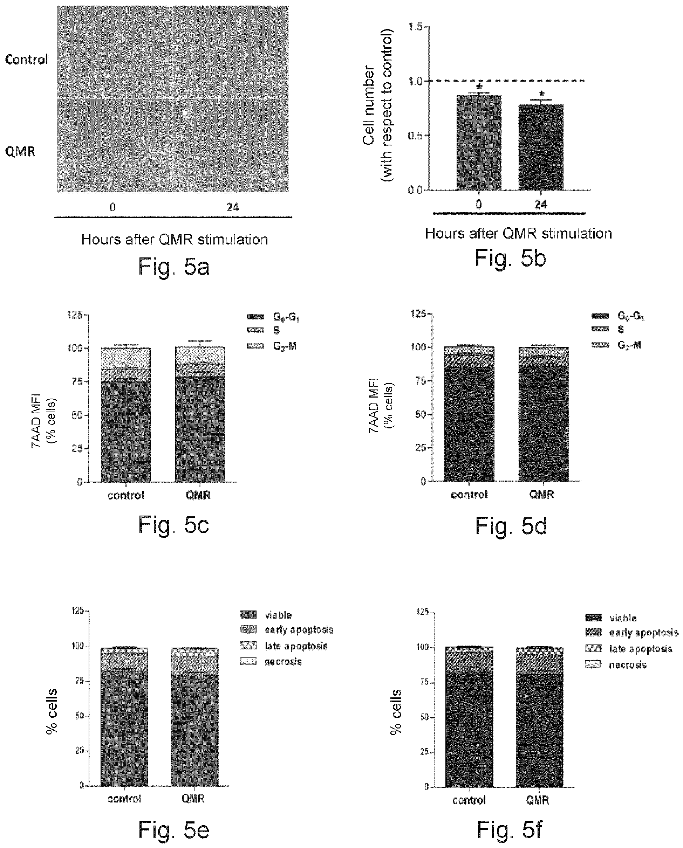

- Figures 5a to 5f show the effects of the QMR stimulation on mesenchymal stromal cells (BM-MSC): Fig. 5a shows representative images, acquired with an Axiovert 40 CFL inverted light microscope, 10X magnification, of BM-MSC cell cultures at 0 and 24 hours after the end of a 24-hour QMR stimulation and of control cells (Control); Fig. 5b shows the viability of BM-MSC cells at 0 and 24 hours after the end of a QMR stimulation for a time of 24 hours compared to control cells, obtained by means of a test with Trypan blue; Figures 5c-5f show the percentage of BM-MSC cells for each phase of the cell cycle (G₀-G₁, S, G₂-M) and the induction of apoptosis by flow cytometry at 0 and 24 hours after the end of the QMR stimulation (5c/5e and 5d/5f, respectively); the results are expressed as mean ± SEM of at least 3-4 independent experiments; * = p <0.05; QMR Vs Control;

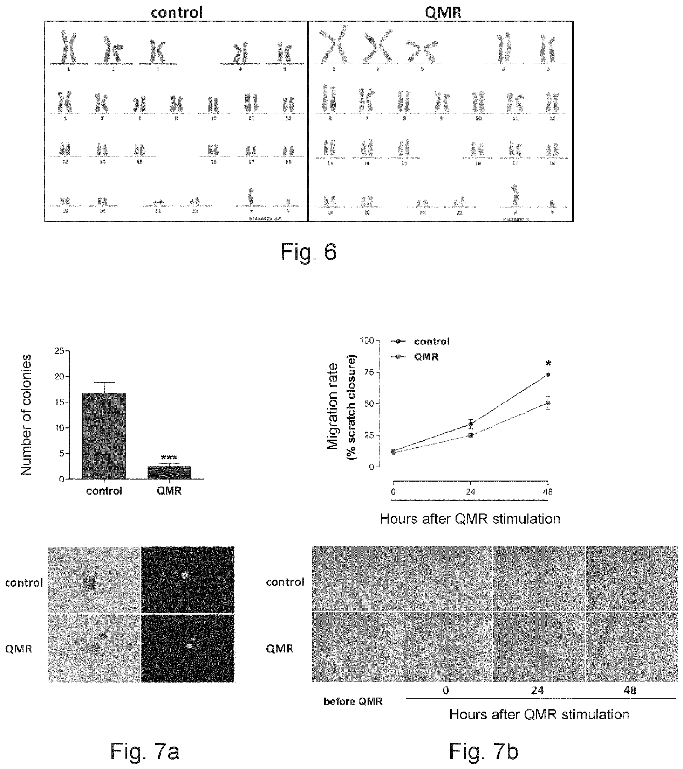

- Figure 6 shows representative images of the karyotype of BM-MSC cells under basal conditions (Control) and after QMR stimulation; chromosomes in metaphase were detected by GTG method; the images are representative of 3 independent experiments;
- Figures 7a and 7b show the aggressiveness and invasiveness of A172 cells after QMR stimulation: in Fig. 7a, A172 cell colonies were labelled with calcein and counted using an Axiovert CFL inverted light microscope; in Fig. 7b A172, cells were grown in silicon inserts placed directly inside the Petri dishes and, after QMR stimulation, the migration rate was assessed up to 48 hours after the end of the stimulation using an inverted light microscope, where the percentage of scratch closure indicative of the cell migration rate was quantified using Image J software; the results are expressed as mean ± SEM of at least 3-5 independent experiments; * = p <0.05; *** = p <0.001; QMR Vs Control;

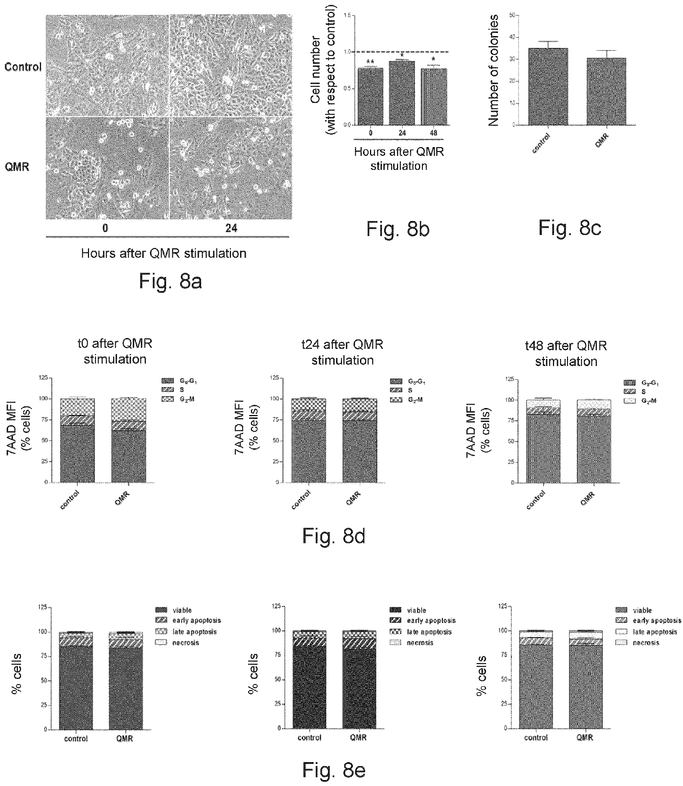

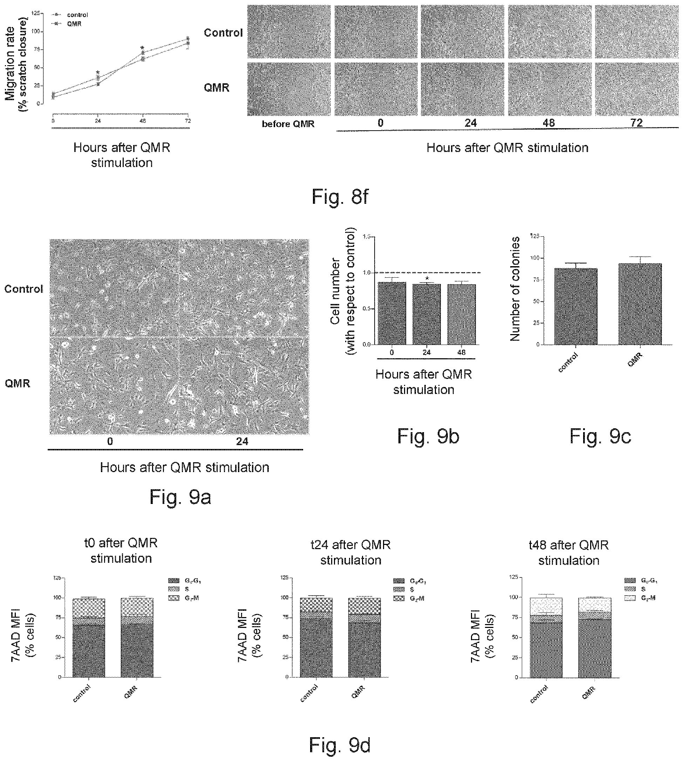

- Figures 8a to 8f show the effects of QMR stimulation on T98G glioblastoma cells: Fig. 8a shows the images acquired with the Axiovert 40 CFL inverted light microscope, 5X magnification, representing cultures of T98G cells at 0 and 24 hours after the end of a 24-hour QMR stimulation and of control cells (Control); Fig. 8b shows the viability of T98G cells at 0, 24 and 48 hours after the end of a 24-hour QMR stimulation compared to control cells, obtained by testing with Trypan blue; Fig. 8c shows the colonies of T98G cells labelled with calcein and counted by means of an Axiovert CFL inverted light microscope; Figs. 8d and 8e show the percentages of T98G cells for each phase of the cell cycle (G₀-G₁, S, G₂-M) and the induction of apoptosis obtained by flow cytometry at 0, 24 and 48 hours after the end of the QMR stimulation; in Fig. 8f, T98G cells were grown in silicon inserts placed directly inside Petri dishes and, after QMR stimulation, the migration rate up to 72 hours after the end of the stimulation was evaluated by inverted light microscope; the percentage of scratch closure indicative of the cell migration rate was quantified using Image J software; the results are expressed as mean ± SEM of at least 3-5 independent experiments; * = p <0.05; ** = p <0.01; QMR Vs Control;

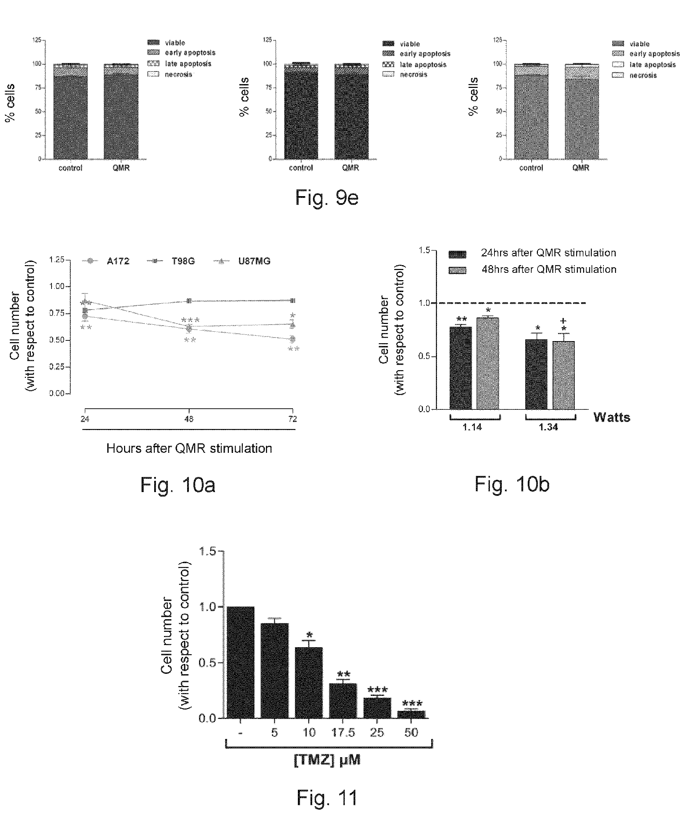

- Figures 9a to 9e show the effects of the QMR stimulation on U87MG glioblastoma cells: Fig. 9a shows images acquired with the Axiovert 40 CFL inverted light microscope, 5X magnification, representative of U87MG cell cultures at 0 and 24 hours after the end of a 24-hour QMR stimulation and of control cells (Control); Fig. 9b shows the viability of U87MG cells at 0, 24 and 48 hours after the end of a 24-hour QMR stimulation compared to the control cells, obtained by means of a test with Trypan blue; in Fig. 9c colonies of U87MG cells were labelled with calcein and counted by Axiovert CFL inverted light microscope; Figs. 9d and 9e show the percentages of U87MG cells for each phase of the cell cycle (G₀-G₁, S, G₂-M) and the induction of apoptosis obtained by flow cytometry at 0, 24 and 48 hours after the end of the QMR stimulation; the results are expressed as mean ± SEM of at least 3-5 independent experiments; * = p <0.05; ** = p <0.01; QMR Vs Control;
- Figures 10a and 10b show the cell viability values for A172, T98G and U87MG cells after 24 and 48 hours of QMR stimulation; in Fig. 10b, T98G cells were stimulated with QMR at a power 15% higher than the other cell lines and the proliferation rate was evaluated after 24-48 hours of QMR stimulation; the results are expressed as mean ± SEM of at least 1-4 independent experiments; * = p <0.05; ** = p <0.01; *** = p <0.001; QMR Vs Control;
- Figure 11 shows the cell viability values for A172 cells treated with the drug temozolomide (TMZ) for a time of 144 hours; the results were obtained by means of a test with Trypan blue and are expressed as mean ± SEM of at least 3 independent experiments; * = p <0.05; ** = p <0.01; *** = p <0.001; TMZ Vs Control (One-sample t-test);

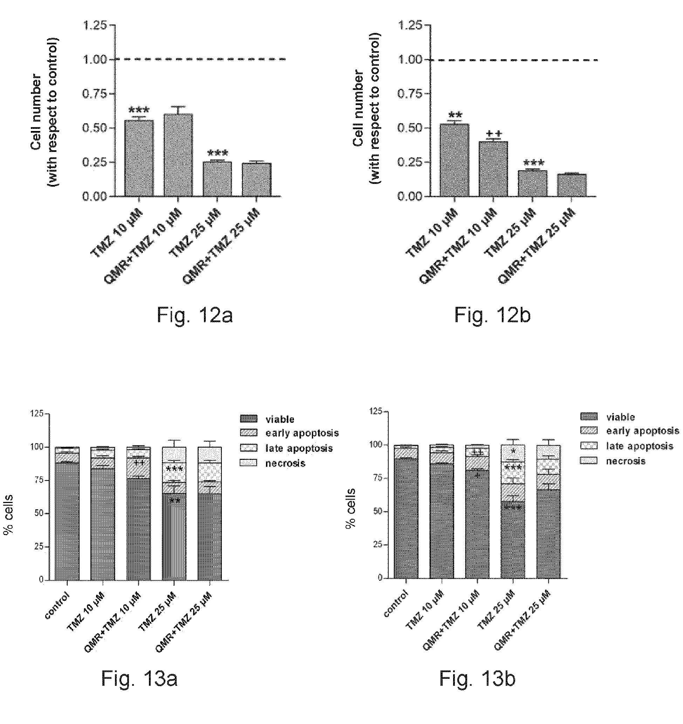

- Figures 12a and 12b show the effects of the combination of treatment with TMZ drug and QMR stimulation on A172 cells; TMZ was administered for 144 hours, following or simultaneously with 24-hour QMR stimulation, where cell viability was monitored by means of a test with Trypan blue and the results are expressed as mean ± SEM of at least 3-5 independent experiments; * = p <0.05; ** = p <0.01; *** = p <0.001; Treatment Vs Control; + = p <0.05; ++ = p <0.01; +++ = p <0.001 for combination TMZ and QMR Vs TMZ alone;
- Figures 13a and 13b show the values of apoptosis induction on A172 cells treated with TMZ (10µM and 25µM) after (Fig. 12a) or simultaneously (Fig. 12b) with the QMR stimulation, where the values were obtained by flow cytometry; the results are expressed as mean ± SEM of at least 4-6 independent experiments; * = p <0.05; ** = p <0.01; *** = p <0.001 for Treatment Vs Control; + = p <0.05; ++ = p <0.01; +++ = p <0.001 for combination TMZ and QMR Vs TMZ alone;

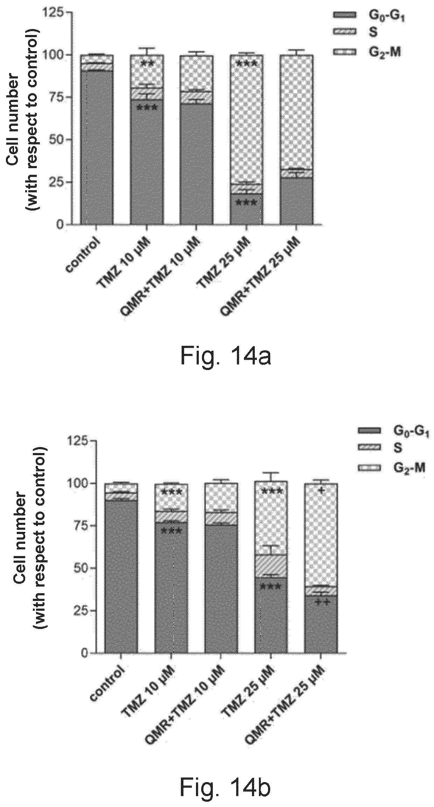

- Figures 14a and 14b show the percentages of A172 cells for each phase of the cell cycle (G₀-G₁, S, G₂-M): cell cycle progression was monitored by flow cytometry of TMZ-treated cells after QMR stimulation (Fig. 14a), and simultaneously with QMR stimulation (Fig. 14b); the results are expressed as the mean ± SEM of at least 3-5 independent experiments; * = p <0.05; ** = p <0.01; *** = p <0.001 for TMZ Treatment and QMR Vs Control; + = p <0.05; ++ = p <0.01; +++ = p <0.001 for TMZ Treatment and QMR Vs TMZ alone.

### Detailed description

As indicated above, the present description relates to a non-claimed method for blocking or inhibiting the growth of neoplastic cells in a target region of a living organism.

The term neoplastic cells is used in this document to refer to those cells that escape the proliferation control mechanisms and follow their own autonomous program of reproduction. They form a neoplasm, i.e. an abnormal mass of cells whose growth is excessive and uncoordinated compared to that of normal cells, and which progresses excessively even after the stimuli that evoked it have ceased.

The aforesaid neoplasm, and optionally the region of the body of the living organism where this tumor mass expands, also represents the target region mentioned above.

By way of example not to be considered limiting, this neoplasm may include lung, colorectal, mammary, prostate, pancreas, liver neoplasm, or haematological neoplasms of bone marrow cells, lymphatic system, immune system, etc.

In particular, the neoplastic cells of the present description comprise glioblastoma (GBM) cells, a malignant astrocytic tumor, and the target region comprises the central nervous system.

The method of the description provides for treating the aforesaid neoplastic cells by applying electric current waves to the aforesaid target region for a predefined period of time, wherein said electric current waves have a preferably sinusoidal waveform with a fundamental frequency higher than or equal to 2 MHz.

The application of current waves having a sinusoidal waveform with a fundamental frequency higher than or equal to 2 MHz transmits energy to the molecules to which these current waves are applied, which corresponds to the so-called "molecular resonance", defined by the term Quantum Molecular Resonance (QMR).

It is known from document EP1087691 and from document *Pozzato G et al, 2003,* on behalf of the Applicant, that this QMR energy is just sufficient to break the bonds among the molecules hit by the passage of current, making it particularly useful when applied to a scalpel. This scalpel is in fact able to cut the regions of interest, without producing any effect of rupture, tear, necrosis, decrease or increase in the thickness of the tissues involved, alteration of the liquid content, coagulation or other degenerative effect around the cut.

However, it has now been surprisingly discovered that current waves having a waveform with a fundamental frequency higher than or equal to 2 MHz perform an antitumor action when applied to cells of the neoplastic type.

The Applicants have in fact discovered that the application of electric current waves, such as to transmit the QMR resonance energy to the neoplastic cells, allows to obtain the reduction of the motility and aggressiveness of the aforesaid cells, decreasing their ability to migrate through the matrices, for example, to give rise to new tumor lesions.

Experiments on BM-MSC cells have also highlighted that the application of such current waves appears to be harmless towards normal, non-neoplastic type cells, thus ensuring that the method of the description is selective in blocking or inhibiting the growth of neoplastic cells only and not the growth of the supporting cells that are found around these neoplastic cells, as is the case, instead with standard type antitumor therapies.

Furthermore, it has been identified that the application of such current waves to glioblastoma cells causes a global alteration of their protein structure which ultimately leads to cell death.

Advantageously, instead, this protein alteration is quickly counteracted in healthy cells.

The use of fundamental frequency values ranging between 2 and 64 MHz, more preferably ranging between 2 and 20 MHz, even more preferably ranging between 4 and 20 MHz, has proved particularly advantageous in the method of the description.

Particularly preferred is the use of electric current waves having a waveform with a fundamental frequency of about 4 MHz.

Preferably, such a waveform is a waveform of the sinusoidal type.

Furthermore, according to one aspect of the method of the description, such a sinusoidal waveform is distorted by the presence of harmonics.

Again, according to another aspect of the method of the present description, the sinusoidal waveform is distorted by the presence of at least second and third order harmonics.

Preferably, the sinusoidal waveform is distorted by the presence of at least first, second and third order harmonics.

As shown by the results of the experiments described below, the application of the aforesaid electric current waves to neoplastic cells makes it possible to block or inhibit the growth of neoplastic cells with a high rate of success, leaving the healthy cells present in the target region substantially unharmed.

The effect of combining the application of such electric current waves with the treatment of neoplastic cells with antitumor drugs of known type was also tested.

The term antitumor drug refers to any molecule or combination of molecules, for pharmaceutical use, intended to inhibit or block the growth and spread of tumor formations.

The use of the antitumor drug temozolomide (TMZ) is particularly preferred.

It was surprisingly found that the combination of applying such electric current waves having a waveform with a fundamental frequency higher than or equal to 2 MHz, preferably ranging between 2 and 64 MHz, more preferably ranging between 2 and 20 MHz, even more preferably ranging between 4 and 20 MHz, much more preferably about 4 MHz, to neoplastic cells of the aforesaid target region for a predefined period of time, either previously, simultaneously or subsequent to the treatment with an antitumor drug, allows obtaining a greater antitumor efficacy of the antitumor drug itself.

In particular, the use of the antitumor drug in combination with QMR allows to obtain a greater reduction in the number of live neoplastic cells than the use of the antitumor drug alone on neoplastic cells.

It is therefore highlighted that the combination of the treatment with an antitumor drug with the application of the aforesaid electric current waves exerts a greater antitumor effect in the treated neoplastic cells than the use of the antitumor drug alone on these neoplastic cells, thus allowing, with equal antitumor effect, to decrease the dosage of the antitumor drug.

Still advantageously, thanks to this reduction in dosage achieved through the use of QMR, it is possible to achieve a substantial reduction in the side effects caused by treatment with the antitumor drug, for the same antitumor effect achieved.

This advantage is particularly evident when the antitumor drug is administered simultaneously with or after the treatment with QMR.

This advantage is even more evident when the drug is administered simultaneously with QMR treatment.

Still advantageously, the combination of treatment with antitumor drug and QMR allows to obtain an increase in the number of cells in early apoptosis.

It is clear that QMR can be used as an effective adjunct to standard chemotherapy therapies to enhance the effects of currently used antitumor drugs, without a corresponding increase in toxicity.

In addition to these antitumor drugs, QMR can also be applied in combination with other types of antitumor treatments.

Some examples, not to be considered limiting, of such antitumor treatments comprise surgical treatments, radiosurgery, ionizing radiation treatments, chemotherapy treatments with alkylating agents, antimetabolites, antitumor antibiotics, topoisomerase inhibitors, antifungal drugs, corticosteroids, biological drugs, differentiating agents, hormones, drugs that stimulate the immune system, monoclonal antibodies.

Therefore, an antitumor drug for use in a method for blocking or inhibiting the growth of neoplastic cells in a target region of a living organism also forms part of the present description, wherein the method comprises treating the neoplastic cells with such antitumor drug simultaneously or after the application of electric current waves to the aforesaid target region for a predefined period of time. As stated above, such electric current waves have a waveform with a fundamental frequency higher than or equal to 2 MHz.

It is not excluded that, according to one aspect of the description, the treatment of the neoplastic cells with the antitumor drug takes place prior to the application of the aforesaid electric current waves.

Preferably, this waveform has a fundamental frequency ranging between 2 and 64 MHz, preferably ranging between 2 and 20 MHz, more preferably ranging between 4 and 20 MHz.

More preferably, this waveform has a fundamental frequency of about 4 MHz. Still preferably, the aforesaid waveform is a sinusoidal waveform.

Additionally, this sinusoidal waveform is distorted by the presence of harmonics.

Preferably, such a sinusoidal waveform is distorted by the presence of at least second and third order harmonics.

According to one aspect of the description, the antitumor drug is used for use in a method comprising treating neoplastic cells with the antitumor drug simultaneously with the application of the aforesaid electric current waves to the target region of a living organism while the neoplastic cells are treated with the antitumor drug.

Returning to the method of the present description, this method preferably comprises the following steps:
- connecting a device adapted to generate electric current waves, having a waveform with a fundamental frequency higher than or equal to 2 MHz and wherein, preferably, this waveform is a sinusoidal waveform distorted by the presence of harmonics, to one or more electrodes;
- applying at least one of these electrodes to the surface of the body of the living organism at which the target region comprising the aforesaid neoplastic cells is located, or implanting at least one of these electrodes in the living organism at or near the target region comprising the aforesaid neoplastic cells;
- activating the device such as to transfer the current waves to these one or more electrodes and keeping the device activated for a predefined time.

Preferably, by a living organism it is intended a human subject.

These electrodes are of a suitable shape and configuration to transmit the aforesaid electric current waves to the target region.

By way of example not to be considered limiting, such electrodes may be of a non-implantable type and, for example, may be in the form of a handpiece or in the form of a suction cup, or again may be essentially laminar in shape and applicable by adhesion to the skin of the subject, or such electrodes may be in the form of a probe.

When the electrodes used in the method of the description have a laminar shape, they are essentially flexible so as to follow the shape of the surface of the body without difficulty and, moreover, they are optionally also equipped with an adhesive substance which eases keeping them in contact with the body during the application of the waveform generated by the device.

It is not excluded that one or more of the aforesaid electrodes may be of the implantable type.

A device for blocking or inhibiting the growth of neoplastic cells in a target region of a living organism therefore also forms part of the present description.

Such a device is usable in the method of the description.

In particular, the device of the description is configured to generate electric current waves having a waveform with a fundamental frequency higher than or equal to 2 MHz.

The target region preferably belongs to a living organism, more preferably a human subject, as already indicated above.

According to a preferred embodiment of the device of the description, it comprises a radiofrequency circuit connected to one or more electrodes and adapted to supply each of these electrodes with a current wave having a fundamental frequency higher than or equal to 2 MHz.

Preferably, the device further comprises a rectifier circuit powered by the mains voltage which supplies preferably continuous voltage, more preferably stabilised, to the aforesaid radiofrequency circuit.

Such a radiofrequency circuit preferably comprises at least one electronic switch which is powered by the voltage and driven by a driving circuit.

More in detail, the radiofrequency circuit presents at its output a current wave with a fundamental frequency higher than or equal to 2 MHz and is adapted to transmit this current wave to these electrodes. Preferably, this fundamental frequency is ranging between 2 and 64 MHz, more preferably ranging between 2 and 20 MHz, even more preferably ranging between 4 and 20 MHz.

More preferably, the fundamental frequency corresponds to about 4 MHz. This current wave also has a sinusoidal shape.

This current wave is also advantageously distorted by the presence of harmonics.

These harmonics are preferably harmonics of at least the second and third order.

More preferably, the current wave has a sinusoidal shape distorted by the presence of at least first, second and third order harmonics.

This current wave also circulates in a preferably broadband resonant circuit on the frequency of the fundamental wave of the distorted sinusoidal shape.

According to the preferred embodiment of the description, the device generates high frequency sinusoidal alternating electric current waves of 4 MHz with harmonics from 4 to 64 MHz.

According to one aspect of the device of the description, the electrodes of said device are configured in such a way that they can transmit the aforesaid current wave to the target region.

It is specified that the electrodes of the device of the description may have a shape and configuration corresponding to the shape and configuration of the electrodes described above for the method of the description.

According to one aspect of the device of the description, such electrodes are of the non-implantable type and are adapted to be applied to the surface of the body of the organism at or near which the target region comprising the neoplastic cells is located.

According to a variant embodiment of the device, it comprises a helmet configured to allow the housing of the aforesaid electrodes and to allow both their connection with the aforesaid radiofrequency circuit and their arrangement at or near the target region.

It should be noted that, according to a particular variant embodiment of the device comprising the aforesaid helmet, the latter is configured to also house the radiofrequency circuit.

According to a further variant of the device, it comprises a cap of the wearable type configured to house the aforesaid electrodes and allow them to be applied at or near the target region.

According to another variant embodiment of the device, it comprises at least one band provided with the aforesaid electrodes. Optionally, this band is configured to also house the device's radiofrequency circuit.

This band is also configured to be placed laterally and/or frontally and/or posteriorly to the head of the subject.

It is not excluded that such a band is configured to be placed at the person's waist and to be worn by the person as a belt.

Furthermore, it is not excluded that this band may be worn by the subject in a different way than indicated above.

According to a particular variant embodiment of the device of the description, at least one or more of the electrodes of the device of the description are electrodes of the implantable type.

The term "implantable" means an electrode intended to be implanted wholly or partially, by surgical or medical intervention, into the human body.

According to another variant embodiment of the device of the description, it is configured so as to be completely implantable.

By way of example, the present implantable device has a configuration substantially similar to the configuration of an implantable pacemaker.

Other aspects and advantages of the present description will appear when reading the following examples, which are to be considered as illustrative and non-limiting.

### Examples

### Example 1. Materials and methods

### 1.1 Cell cultures and cell growth

Glioblastoma cell lines A172, T98G and U87MG were used. A172 and T98G cells (Sigma-Aldrich, St. Louis, MO, USA) were cultured in Dulbecco's Modified Eagle's Medium/Nutrient Mixture F-12 (DMEM/F12 GlutaMAX, Gibco, Thermo Fisher Scientific, Waltham, MA, USA) added with 10% foetal bovine serum (FBS) (Qualified Australian, Gibco, Thermo Fisher Scientific) and 1% penicillin/streptomycin (Sigma-Aldrich).

U87MG cells (courtesy of Prof. Massimo Dominici, Laboratory of Cellular Therapy, University Hospital of Modena and Reggio Emilia Modena, Italy) were cultured in Dulbecco's Modified Eagle's Medium (DMEM) with GlutaMAX (Gibco, Thermo Fisher Scientific) added with 10% FBS (Gibco, Thermo Fisher Scientific) and 1% penicillin/streptomycin (Sigma-Aldrich).

Bone marrow-derived mesenchymal stromal cells (BM-MSC) were also used as non-tumor type control cells. BM-MSC cells were produced in the Advanced Cellular Therapies Laboratory of the Complex Operative Unit of Haematology of ULSS8 Berica as described previously (*Sella et al, 2018*)*.* Briefly, MSC were isolated from cells obtained by washing discarded bone marrow collection bags and filters from healthy donors (Ethics Committee auth. No. 107/18 of 12.02.2019). After washing, cells were centrifuged at 2000 rpm for 10 min and seeded at a density of 1x10⁵ cells/cm² in DMEM with GlutaMAX (Gibco, Thermo Fisher Scientific,) added with 10% FBS (Gibco, Thermo Fisher Scientific) and 1% penicillin/streptomycin (Sigma-Aldrich). Cell cultures were incubated at 37°C in a humid atmosphere with 5% CO₂. Cells that did not adhere were removed 72 hours after seeding and new medium was added. The medium was changed every 3-4 days. At 80% confluence, BM-MSC cells were re-seeded at a density of 2000 cells/cm².

### 1.2 QMR stimulation protocol

The cell lines were stimulated with QMR using a prototype QMR device from the company Telea (Telea Electronic Engineering, Sandrigo, VI, Italy). The device, suitably adapted for the present in vitro experiments, generated high-frequency sinusoidal alternating electric current waves of 4 MHz with harmonics from 4 to 64 MHz and had the following data: power supply 100÷230 V ~ 50/60 Hz; maximum output power, 5 W/400 Ω.

QMR was applied to the cells using a pair of electrodes that were placed inside a 100 mm Petri dish, at the edge, and connected to the generator device. The transmission of electric fields to the medium of the cell cultures generated heat (Joule effect). The average temperature increase in the medium of the stimulated cells was 5°C. This value was measured by means of three independent measurements with a data-logger probe (iLog, Escort Scunthorpe, UK) placed in the culture medium. In order to compensate for the increased temperature of the medium of the stimulated cells and also to ensure an average temperature of 37°C for the stimulated cells, the incubator used for the stimulated cells was set at 32°C at 5% CO₂ and the temperature of the laboratory was maintained between 20°C and 25°C.

### 1.3 Experimental setup

Based on the cell line used, a defined number of cells were seeded in 100 mm plates (Greiner Bio-One, Frickenhausen, DE) in order to be stimulated at 60% confluence. The cells were then exposed to QMR for 24 hours and analysed 0-24-48 hours after the end of the stimulation.

More specifically, the cells were washed with D-PBS (Sigma-Aldrich), detached with 1X TrypLE Select (Gibco, Thermo Fisher Scientific) and the aliquots obtained were used to investigate cell viability, apoptosis, cell cycle, karyotype and proteomics. A cell aliquot was re-seeded for evaluation of cell growth capacity in semi-solid medium (soft agar assay), while cell migration was monitored directly on QMR-stimulated plates before and after stimulation. The effect of the combined use of QMR and the drug TMZ (Sigma-Aldrich) was also tested.

For these analyses, the cells were treated for 144 hours with 10-25 µM of TMZ administered at the same time as or after QMR stimulation. The TMZ stock solution was prepared in DMSO at a final concentration of 10 mM and maintained at 4°C. Subsequent dilutions were made in fresh culture medium and administered to the cells. Cell viability, apoptosis and the cell cycle were assessed after 144 hours.

### 1.4 Cell viability: Trypan blue exclusion assay

After stimulation, the cells were harvested and suspended in a 1:1 ratio with a Trypan blue solution (Gibco, Thermo Fisher Scientific) in culture medium. The cells were counted using a Burker's chamber and the number of live cells was obtained by applying the following formula: [(number of cells x 10,000 x 2 x V)/9], where V is the total volume of cell solution obtained and 2 is the dilution factor of the solution with the dye (2X).

### 1.5 Apoptosis: Labelling with Annexin V/7-aminoactinamycin D (7-AAD)

After stimulation, 1.5 x 10⁵ cells were harvested and centrifuged at 400g for six minutes. After washing with binding buffer, the cells were labelled with Annexin V/7-AAD according to the manufacturer's instructions (Invitrogen, Carlsbad, CA, USA). After dilution with binding buffer, the fluorescence of 2 x 10⁴ cells/sample was detected using an FC500 flow cytometer (Beckman Coulter, Brea, CA, USA). The cell population was separated into four groups: live cells with negative fluorescence for both Annexin V and 7-AAD; cells in early apoptosis positive for Annexin V and negative for 7-AAD (Annexin V+/7-AAD-); cells in late apoptosis with double positivity for Annexin V and 7-AAD (Annexin V+/7-AAD+) and dead cells negative for Annexin V and positive for 7-AAD (Annexin V-/7-AAD+).

### 1.6 Cell cycle

After stimulation, 5 x 10⁵ cells were harvested and centrifuged at 400g for six minutes. The cells were then washed with D-PBS and fixed and permeabilised with acetone (80% solution in water). After one hour at 4°C, the acetone was removed by centrifugation, the cells were washed and labelled with 1 µg/mL with 7-AAD (Invitrogen) for 1 hour at room temperature. The fluorescence of 2.5 x 10⁴ cells/sample was analysed using a FC500 flow cytometer (Beckman Coulter). EXPO 32 software (Coulter Systems, Fullerton, CA, USA) was used to calculate the percentage of cells in the different phases of the cell cycle. Diplode cycles were considered and corrected for cell clusters.

### 1.7 Karyotype analysis

To assess whether QMR stimulation was safe and therefore did not cause chromosome alterations, BM-MSC mesenchymal stromal cells were stimulated for 24 hours with QMR and tested by G-Trypsin-Giemsa banding following standard techniques with 350/400 band resolution. 20 metaphases were analysed and 3 were karyotyped. Unstimulated BM-MSC cells were used as control cells.

### 1.8 Cell migration (scratch test)

The scratch test represents a 2D cell migration approach to semi-quantitatively detect cell motility. The purpose of scratching (opening) is to create a cell-free area (or opening) in order to induce the surrounding cells to migrate and close this opening. The cells were seeded in two-well silicone inserts (IBIDI GmbH, Gräfelfing, DE) that achieve a 500 µm cell-free opening on the cell monolayer. When 60% cell confluence was reached, the insert was removed and the cells were stimulated for 24 hours with QMR. Cell migration was monitored over time by image acquisition before stimulation and 0-24-48 hours after stimulation. Image acquisition was performed with an Axiovert 40 CFL inverted light microscope (Carl Zeiss, Oberkochen, DE). The percentage of closure of the opening was analysed with ImageJ software (National Institutes of Health, Bethesda, MD, USA) and the migratory capacity was assessed by means of a migration rate curve, which was calculated as shown below: Closure % = [(Area tx - Area ty)/ Area tx] x 100, where tx represents the acquisition time and ty the next time point.

### 1.9 Soft Agar

The colony formation assay in semi-solid medium (soft agar) is a method used to monitor cell growth independently of cell anchoring to the substrate and which measures cell proliferation in semi-solid medium by optical colony counting. The rate of colony formation in soft agar changes depending on the cell line used. Therefore, the concentration of agarose, the number of cells seeded and the final day of the experiment were optimised for each cell line. The cell suspension was prepared in a 0.4% agarose solution in cell medium and seeded on a solidified layer of 0.6% agarose in complete medium. After 1 hour at room temperature, 160 µl of complete medium was added and a further 100 µl were added every week until the end of the experiment. The plates were transferred to an incubator at 37°C at 5% CO₂ for 21-24 days before being labelled with 100 µM calcein (Sigma-Aldrich) for 30 minutes. The colonies consisting of at least 50 cells were counted using an Axiovert 40 CFL inverted light microscope (Carl Zeiss).

### 1. 10 Proteomics

1 x 10⁶ cells were harvested and centrifuged at 400g for 6 minutes, washed with D-PBS and lysed with lysis buffer on ice (Pierce^{™} RIPA buffer, Thermo Fisher Scientific) added with protease inhibitor cocktail (Cell Signalling, Danvers, MA, USA). After 30 minutes on ice, the cell lysates were centrifuged at 14000g for 10 minutes at 4°C and the protein supernatant was determined colorimetrically by BCA assay (Pierce^{™} BCA Protein Assay Kit, Thermo Fisher Scientific). Bovine serum albumin was used as the standard (Sigma Aldrich).

### 1.11 Liquid chromatography - mass spectrometry

### Protein extraction

Liquid chromatography - mass spectrometry (LC/MS-MS) is an analytical technique that combines the separating capacity of liquid chromatography with the high sensitivity and selectivity of the mass analysis of the triple quadrupole mass spectrometry. For sample preparation, 50 µg of protein lysate were precipitated with acetone and the obtained protein pellet was dissolved in a 6M solution of urea and 100 mM ammonium bicarbonate (pH 8). Samples were reduced using 10 mM dithiothreitol for 1 hour at room temperature and alkylated with 20 mM iodoacetamide in the dark for 30 minutes at room temperature. Subsequently, the proteins were subjected to digestion with the endopeptidase enzyme Lys-C (Promega) at an enzyme/protein ratio of 1:100 (w/w) for 3 hours at room temperature. The proteins were then diluted 4 times in 50 mM ammonium bicarbonate and digested overnight with trypsin (Promega) 1:100 (w/w) at room temperature. Proteolysis was interrupted by the addition of 1% trifluoroacetic acid. Samples were then desalinated, vacuum dried and resuspended in 0.1% formic acid for LC-MS/MS analysis.

### Protein identification through specific databases and relative quantification

The samples were analysed using the Easy-nLC 1200 system (Thermo Fisher), coupled in-line with an Orbitrap Fusion Tribrid mass spectrometer (Thermo Fisher). A reversed-phase column (Acclaim PepMap RSLC C18, 2µm particle size, 100Å pore size, id 75 µm, Thermo Fisher Scientific) with a two-component mobile phase was used to separate the digested peptides: 0.1% formic acid in water (buffer A) and 0.1% formic acid in acetonitrile (buffer B). Peptides were eluted using a gradient from 5% to 25% for 52 minutes, followed by 25% to 40% for 8 minutes and finally by 40% to 98% for 10 minutes at a flow rate of 400 nL/min. The data-dependent acquisition (DDA) method is based on full scans performed at a resolution power of 120000 fwhm (full width at half maximum) (at 200 m/z), and a maximum injection time of 50 ms. The mass range of 350-1100 m/z was examined for the precursors, with the first mass set at 140 m/z for the fragments. Full scans were followed by a series of MS/MS (HCD) scans for a cycle time of 3 seconds, at a collision energy of 30% and detected in the ion trap with a maximum injection time of 150 ms. The dynamic exclusion time was set at 50 sec. Unprocessed data were searched in Proteome Discoverer 2.2 software (Thermo Fisher Scientific). Peptide searches were carried out using the Human protein FASTA file (uniprot). The proteins were identified with the MASCOT search engine (Matrix Science inc, Boston, MA, USA) using a precursor mass tolerance of 10 ppm and a product mass tolerance of 0.6 Da.

### 1.12 Bioinformatic analysis

The different abundances of proteins expressed in the different experimental groups (at t0 and t24, respectively) were used to perform a hierarchical cluster analysis using the Clustvis tool (Metsalu T et al, 2015). Gene Ontology (GO) and Pathway annotation of protein IDs was performed using the EnrichR gene set enrichment analysis server (http://amp.pharm.mssm.edu/Enrichr/), by applying biological processes and *Reactome* categorisation with the significance threshold set at p <0.05. The network of protein interactions was constructed using the STRING interaction database, version 11.0 (https://string-db.org/) (Mering C et al, 2003).

### 1.13 Statistical analysis

All data were analysed using GraphPad program (GraphPad Software, San Diego, CA, USA) and were expressed as mean ± standard error. The One-sample t-test was used for the analysis of the results expressed as a ratio/percentage of the control (e.g. trypan blue exclusion assay). The unpaired Student's t-test was used for all the other analyses. Data with p < 0.05 were considered as significant.

### Example 2. Results

### 2.1 QMR treatment reduces the growth of glioblastoma cells by modifying the cell cycle progression

The effects of QMR treatment on A172 glioblastoma cells were assessed by analysing cell morphology, proliferation rate, apoptosis and cell cycle of the cells immediately at the end of the treatment and 24 and 48 hours after the end of QMR stimulation. The compliance with these timeframes made it possible to obtain an assessment of the permanence of the effects obtained with the treatment and the ability of the treated cells to restore their cellular functions to pre-treatment levels.

It was surprisingly found that QMR treatment changes the morphology of treated A172 cells compared to untreated control cells. QMR-treated cells appeared in fact more swollen and more granular than control cells, indicating that cell damage had occurred.

This damage was also found to be persistent over time, as shown in Figure 1.

The cell proliferation rate was also estimated by testing with Trypan Blue. 24 hours after treatment, QMR-treated cells showed a reduction in tumor growth by about 28%. Furthermore, the number of live cells tended to decrease even 24-48 hours after the treatment, suggesting an effect of QMR treatment on the cell cycle progression, as visible in Figure 2.

In support of this surprising finding, the cell cycle of A172 cells was also evaluated using flow cytometry experiments. The results, shown in Figures 3a and 3c, showed that QMR treatment significantly reduces the rate of dividing cells (defined as the percentage of cells in S phase) while the cells in G₂/M phase significantly increase. These results were also confirmed up to 48 hours after the end of the treatment, showing that cell cycle arrest had occurred in the G₂/M phase.

Further, the efficacy of treating cells with QMR was confirmed by the clear apoptotic activation in the treated cells. The number of live cells after QMR treatment was in fact significantly lower (p<0.01) than the number of control live cells not treated with QMR, as shown by the results in Figures 4b and 4c. This reduction was also correlated with an increase of about 10% in the number of cells in early apoptosis and a slight increase in the number of cells in late apoptosis.

### 2.2 QMR treatment does not cause cytotoxic and genotoxic effects on BM-MSC cells

The effects of QMR treatment on healthy BM-MSC cells were tested in order to verify the safety of QMR treatment on non-neoplastic type cells. At the end of QMR stimulation and 24 hours after the end of the treatment, cell morphology, proliferation rate, apoptosis, cell cycle and karyotype were evaluated in treated BM-MSC cells and in untreated control cells. Although a slight decrease in cell growth was displayed at the end of the QMR treatment, as shown in Figure 5b, this effect is not to be considered comparable to that obtained in the treated A172 glioblastoma cells, shown in Figure 2.

Furthermore, BM-MSC cells treated with QMR showed no differences in cell morphology, cell cycle modulation and apoptosis, as visible in Figures 5a, 5c/5d, 5e/5f, respectively.

Furthermore, time did not influence the final effect obtained, as demonstrated by the results obtained which are substantially superimposable at 0 and 24 hours after treatment.

The analysis of the karyotype of BM-MSC cells treated with QMR also showed that the QMR treatment advantageously causes no changes in the number of chromosomes or in their structure, as can be seen in Figure 6.

### 2.3 QMR treatment reduces aggressiveness and invasiveness of A172 glioblastoma cells

It is well known that neoplastic type cells are characterized by uncontrolled proliferation and migration. The ability of cells to grow in semi-solid medium and their mobility rate are therefore considered markers of tumor aggressiveness and invasiveness. Cell colony formation in soft agar and cell migration were therefore analysed under basal conditions and after QMR treatment. The results, shown in Figure 7a, surprisingly showed that the QMR treatment significantly reduces the growth of A172 cells in soft agar. In fact, the number of cell colonies after the QMR treatment was about 85% lower than the number of colonies of untreated control cells. Surprisingly, moreover, the cell migration rate of the A172 cells treated with QMR was also lower in a timedependent manner than that of control cells, as shown in Figure 7b.

### 2.4 QMR treatment modulates the proteomic fingerprint of A 172 glioblastoma cell line

The proteomic profile of A172 and BM-MSC cells treated with QMR was also investigated. The analysis was conducted at the end of the QMR stimulation (time 0) and 24 hours after the end of the treatment. Untreated A172 and BM-MSC cells were used as control groups.

A total of 5104 proteins were identified in the A172 cell line, of which 311 and 308 were found to be significantly altered after QMR treatment at time zero and 24 hours after the end of the treatment, respectively. Cluster analysis, not shown in the figures, highlighted two separate groups of proteins at both testing timeframes, demonstrating that QMR treatment interferes with neoplastic cell activity.

The proteins were found to be differentiated between treated and untreated cells. Specifically, at time zero, 111 proteins were found to be significantly up-regulated, while 200 proteins were found to be down-regulated. Instead, 24 hours after the end of the treatment, 103 up-regulated proteins and 205 down-regulated proteins were found.

More specific analyses of gene ontology and pathway enrichment showed that up-regulated proteins were strongly associated with stress response mechanisms, protein folding and extracellular matrix modelling, suggesting that QMR treatment also acts as a toxic-protein stimulus.

More specifically, QMR-treated cells, compared to untreated control cells, had undergone a significant down-regulation of key factors involved in protein translation, RNA processing and cell cycle involved pathways, leading to cell cycle arrest and death of treated cells.

Advantageously, the effects of the QMR stimulation on BM-MSC cells provided completely different results compared to A172 glioblastoma cells. At the end of QMR treatment, the activation of heat shock proteins was associated with a remodelling of the extracellular matrix. However, this alteration was found to be totally normalised 24 hours after treatment. Therefore, QMR treatment does not interfere with the proliferation rate of BM-MSC cells, supporting the selectivity of QMR treatment towards neoplastic type cells.

### 2.5 QMR treatment on the T98G and U87MG cell lines

The experiments described above were also performed on glioblastoma cell lines that were more aggressive than the A172 cell line cells. These more aggressive cell lines comprise T98G and U87MG cells. Although the proliferation rate was found to be reduced in both cell lines following QMR treatment, there were no significant differences in apoptosis activation, cell cycle progression, clonogenic capacity and cell motility, as shown in Figures 8a to 8f.

Cell viability was also tested after 48 and 72 hours of QMR treatment. Surprisingly, the cell proliferation rate for both A172 and U87MG cells was found to be significantly reduced over time.

Even a 15% increase in treatment power has been shown to affect the rate of cell proliferation. In fact, the cell viability of the QMR-treated cells at higher power was found to be decreased by up to 35%, as shown in the figures.

### 2.6 Application of QMR enhances the effects of the drug Temozolomide (TMZ) in A172 glioblastoma cells

Standard therapies for patients with glioblastoma include, to date, total resection of the lesion, followed by radiotherapy and chemotherapy with the drug TMZ.

Disadvantageously, the efficacy of the drug is limited by the severity of the side effects caused by the drug itself and by the establishment of drug resistance mechanisms. It is therefore essential to find new therapeutic strategies to be used in combination with TMZ in order to lower the dosage thereof and improve the patient's quality of life.

The efficacy of the combined therapy of TMZ and QMR treatment on A172 glioblastoma cells was therefore tested. These cells were treated with 10-25 µM of TMZ at the same time as or after the QMR treatment. Cell viability, apoptosis and cell cycle values were analysed. The TMZ drug concentration was selected on the basis of its IC50 (Figure 14).

The results obtained have surprisingly shown that, when the TMZ drug at a concentration of 10 µM was used in combination with QMR treatment, cell viability was reduced by about 12% more than the reduction in cell viability obtained in cells treated with TMZ drug alone, as shown in Figure 12b.

Furthermore, these data were found to be surprisingly correlated with the data on cell apoptosis. Indeed, the combined administration of TMZ and QMR showed that the reduction in the number of live cells is associated with an increase in the number of cells in early apoptosis, as shown in the graphs in Figures 13a and 13b.

The simultaneous exposure of the cells to the drug TMZ 25 µM and to QMR activates a greater alteration in cell cycle progression, compared to cells treated with the TMZ drug alone.

These data surprisingly demonstrate that the combined therapeutic strategy of TMZ with QMR induces significant cell cycle arrest in the G2-M phase and reduces the percentage of cells in the G0-G1 phase, as shown in Figure 14b.

### References

*1.* L. M. DeAngelis, Brain tumors. N Engl J Med 344, 114-123 (2001*).*
*2.* C. Alifieris, D. T. Trafalis, Glioblastoma multiforme: Pathogenesis and treatment. Pharmacology & therapeutics 152, 63-82 (2015*).*
*3.* T. A. Wilson, M. A. Karajannis, D. H. Harter, Glioblastoma multiforme: State of the art and future therapeutics. Surg Neurol Int 5, 64-64 (2014*).*
*4.* K. P. Becker, J. Yu, Status quo--standard-of-care medical and radiation therapy for glioblastoma. Cancer journal (Sudbury, Mass.) 18, 12-19 (2012*).*
*5.* Q. T. Ostrom et al., The epidemiology of glioma in adults: a "state of the science" review. Neuro-Oncology 16, 896-913 (2014*).*
*6.* M. O. Mattsson, M. Simkó, Emerging medical applications based on nonionizing electromagnetic fields from 0 Hz to 10 THz. Medical devices (Auckland, N.Z.) 12, 347-368 (2019*).*
*7.* F. Pasi et al., Pulsed Electromagnetic Field with Temozolomide Can Elicit an Epigenetic Pro-apoptotic Effect on Glioblastoma T98G Cells. Anticancer Res 36, 5821-5826 (2016*).*
*8.* M. Giladi et al., Mitotic Spindle Disruption by Alternating Electric Fields Leads to Improper Chromosome Segregation and Mitotic Catastrophe in Cancer Cells. Scientific Reports 5, 18046 (2015*).*
*9.* G. Pozzato and G. Vignato, Teoria della risonanza quantica molecolare nella realizzazione del bisturi elettronico "Vesalius". Quintessence International (2003); 5/6:153-155*.*
*10.* S. Sella et al., In-vitro analysis of Quantum Molecular Resonance effects on human mesenchymal stromal cells. PLoS One 13, e0190082 (2018*).*

The present invention is set out in the claims that follow.

## Claims

1. An antitumor drug for use in a method for blocking or inhibiting the growth of neoplastic cells in a target region of a living organism, said method comprising treating said neoplastic cells with said antitumor drug simultaneously with the application of electric current waves to said target region for a predefined period of time while said neoplastic cells are treated with said antitumor drug, said electric current waves having a waveform with a fundamental frequency of about 4 MHz distorted by the presence of at least second and third order harmonics.

2. The antitumor drug according to claim 1, **characterized in that** said waveform is a sinusoidal waveform.

3. The antitumor drug according to any one of the preceding claims, **characterized in that** said antitumor drug comprises temozolomide.

4. The antitumor drug according to any one of the preceding claims, **characterized in that** said neoplastic cells comprise glioblastoma cells and said target region comprises the central nervous system.

5. The antitumor drug for use in a method for blocking or inhibiting the growth of neoplastic cells in a target region of a living organism, said method comprising treating said neoplastic cells with said antitumor drug simultaneously with applying electric current waves to said target region for a predefined period of time, said electric current waves having a waveform with a fundamental frequency of about 4 MHz distorted by the presence of at least second and third order harmonics.

6. The antitumor drug for use in a method for blocking or inhibiting the growth of neoplastic cells in a target region of a living organism, said method comprising treating said neoplastic cells with said antitumor drug prior to applying electric current waves to said target region for a predefined period of time, said electric current waves having a waveform with a fundamental frequency of about 4 MHz distorted by the presence of at least second and third order harmonics.

7. A device configured to block or inhibit the growth of neoplastic cells in a target region of a living organism, said device being configured to generate electric current waves having a waveform with a fundamental frequency of about 4 MHz distorted by the presence of at least second and third order harmonics; said device comprising one or more electrodes connected to at least one radiofrequency circuit adapted to supply each of said electrodes with a current wave having a fundamental frequency of about 4 MHz distorted by the presence of at least second and third order harmonics,
**characterized in that** said one or more electrodes are electrodes of the implantable type, said one or more electrodes being adapted to be implanted at or near said target region.

8. The device according to claim 7, **characterized in that** said waveform is a sinusoidal waveform.

## Patentansprüche

1. Antitumor-Medikament zur Verwendung in einem Verfahren zum Blockieren oder Hemmen des Wachstums von neoplastischen Zellen in einer Zielregion eines lebenden Organismus, wobei das besagte Verfahren das Behandeln der besagten neoplastischen Zellen mit dem besagten Antitumor-Medikament und gleichzeitig das Anwenden von elektrischen Stromwellen auf die besagte Zielregion für einen vordefinierten Zeitraum umfasst, während die besagten neoplastischen Zellen mit dem besagten Antitumor-Medikament behandelt werden, wobei die besagten elektrischen Stromwellen eine Wellenform mit einer Grundfrequenz von etwa 4 MHz aufweisen, die durch das Vorhandensein von Oberwellen mindestens zweiter und dritter Ordnung verzerrt ist.

2. Antitumor-Medikament nach Patentanspruch 1, **dadurch gekennzeichnet, dass** die besagte Wellenform eine sinusförmige Wellenform ist.

3. Antitumor-Medikament nach jeglichem der vorhergehenden Patentansprüche, **dadurch gekennzeichnet, dass** das besagte Antitumor-Medikament Temozolomid umfasst.

4. Antitumor-Medikament nach jeglichem der vorhergehenden Patentansprüche, **dadurch gekennzeichnet, dass** die besagten neoplastischen Zellen Glioblastomzellen umfassen und die besagte Zielregion das zentrale Nervensystem umfasst.

5. Antitumor-Medikament zur Verwendung in einem Verfahren zum Blockieren oder Hemmen des Wachstums von neoplastischen Zellen in einer Zielregion eines lebenden Organismus, wobei das besagte Verfahren das Behandeln der besagten neoplastischen Zellen mit dem besagten Antitumor-Medikament und gleichzeitig das Anwenden von elektrischen Stromwellen auf die besagte Zielregion für einen vordefinierten Zeitraum umfasst, wobei die besagten elektrischen Stromwellen eine Wellenform mit einer Grundfrequenz von etwa 4 MHz aufweisen, die durch das Vorhandensein von Oberwellen mindestens zweiter und dritter Ordnung verzerrt ist.

6. Antitumor-Medikament zur Verwendung in einem Verfahren zum Blockieren oder Hemmen des Wachstums von neoplastischen Zellen in einer Zielregion eines lebenden Organismus, wobei das besagte Verfahren das Behandeln der besagten neoplastischen Zellen mit dem besagten Antitumor-Medikament vor dem Anwenden von elektrischen Stromwellen auf die besagte Zielregion für einen vordefinierten Zeitraum umfasst, wobei die besagten elektrischen Stromwellen eine Wellenform mit einer Grundfrequenz von etwa 4 MHz aufweisen, die durch das Vorhandensein von Oberwellen mindestens zweiter und dritter Ordnung verzerrt ist.

7. Vorrichtung, die konfiguriert ist, um das Wachstum von neoplastischen Zellen in einer Zielregion eines lebenden Organismus zu blockieren oder zu hemmen, wobei die besagte Vorrichtung konfiguriert ist, um elektrische Stromwellen mit einer Wellenform mit einer Grundfrequenz von etwa 4 MHz zu erzeugen, die durch das Vorhandensein von Oberwellen mindestens zweiter und dritter Ordnung verzerrt ist; wobei die besagte Vorrichtung eine oder mehrere Elektroden umfasst, die mit mindestens einer Hochfrequenzschaltung verbunden ist/sind, die dazu ausgelegt ist, jede der besagten Elektroden mit einer Stromwelle mit einer Grundfrequenz von etwa 4 MHz zu versorgen, die durch das Vorhandensein von Oberwellen mindestens zweiter und dritter Ordnung verzerrt ist,
**dadurch gekennzeichnet, dass** die besagte eine oder die besagten mehreren Elektroden vom implantierbaren Typ ist/sind, wobei die besagte eine oder die besagten mehreren Elektroden vom implantierbaren Typ dazu ausgelegt ist/sind, in oder in der Nähe der besagten Zielregion implantiert zu werden.

8. Vorrichtung nach Patentanspruch 7, **dadurch gekennzeichnet, dass** die besagte Wellenform eine sinusförmige Wellenform ist.

## Revendications

1. Médicament anticancéreux destiné à être utilisé dans une méthode visant à bloquer ou à inhiber la croissance de cellules néoplasiques dans une région cible d'un organisme vivant, ladite méthode comprenant le traitement desdites cellules néoplasiques avec ledit médicament anticancéreux simultanément à l'application d'ondes de courant électrique à ladite région cible pendant une période de temps prédéfinie alors que lesdites cellules néoplasiques sont traitées avec ledit médicament anticancéreux, lesdites ondes de courant électrique ayant une forme d'onde avec une fréquence fondamentale d'environ 4 MHz déformée par la présence d'au moins des harmoniques de deuxième et de troisième ordre.

2. Médicament anticancéreux selon la revendication 1, **caractérisé en ce que** ladite forme d'onde est une forme d'onde sinusoïdale.

3. Médicament anticancéreux selon l'une quelconque des revendications précédentes, **caractérisé en ce que** ledit médicament anticancéreux comprend le témozolomide.

4. Médicament anticancéreux selon l'une quelconque des revendications précédentes, **caractérisé en ce que** lesdites cellules néoplasiques comprennent des cellules de glioblastome et que ladite région cible comprend le système nerveux central.

5. Médicament anticancéreux utilisé dans une méthode visant à bloquer ou à inhiber la croissance de cellules néoplasiques dans une région cible d'un organisme vivant, ladite méthode comprenant le traitement desdites cellules néoplasiques avec ledit médicament anticancéreux simultanément à l'application d'ondes de courant électrique à ladite région cible pendant une période de temps prédéfinie, lesdites ondes de courant électrique ayant une forme d'onde avec une fréquence fondamentale d'environ 4 MHz déformée par la présence d'au moins des harmoniques de deuxième et de troisième ordre.

6. Médicament anticancéreux utilisé dans une méthode visant à bloquer ou à inhiber la croissance de cellules néoplasiques dans une région cible d'un organisme vivant, ladite méthode comprenant le traitement desdites cellules néoplasiques avec ledit médicament anticancéreux avant l'application d'ondes de courant électrique à ladite région cible pendant une période de temps prédéfinie, lesdites ondes de courant électrique ayant une forme d'onde avec une fréquence fondamentale d'environ 4 MHz déformée par la présence d'au moins des harmoniques de deuxième et de troisième ordre.

7. Dispositif configuré pour bloquer ou inhiber la croissance de cellules néoplasiques dans une région cible d'un organisme vivant, ledit dispositif étant configuré pour générer des ondes de courant électrique ayant une forme d'onde avec une fréquence fondamentale d'environ 4 MHz déformée par la présence d'au moins des harmoniques de deuxième et de troisième ordre; ledit dispositif comprenant une ou plusieurs électrodes connectées à au moins un circuit de radiofréquence adapté pour fournir à chacune desdites électrodes une onde de courant ayant une fréquence fondamentale d'environ 4 MHz déformée par la présence d'au moins des harmoniques de deuxième et de troisième ordre,
**caractérisé en ce que** lesdites une ou plusieurs électrodes sont des électrodes de type implantable, lesdites une ou plusieurs électrodes étant adaptées pour être implantées au niveau ou à proximité de ladite région cible.

8. Dispositif selon la revendication 7, **caractérisé en ce que** ladite forme d'onde est une forme d'onde sinusoïdale.
